# EUROPEAN PATENT SPECIFICATION

(11) **EP 3 677 173 B1**
(45) Date of publication and mention of the grant of the patent: **28.09.2022**
(21) Application number: 19305010.1
(22) Date of filing: 04.01.2019
(51) Int. Cl.: A61B 5/022

(54) **CUFF-TYPE MONITORING DEVICE FOR MONITORING CARDIOVASCULAR PARAMETERS**
MANSCHETTENARTIGE ÜBERWACHUNGSVORRICHTUNG ZUR ÜBERWACHUNG VON KARDIOVASKULÄREN PARAMETERN
DISPOSITIF DE SURVEILLANCE DE TYPE À BRASSARD POUR SURVEILLER LES PARAMÈTRES CARDIO-VASCULAIRES

(43) Date of publication of application: 08.07.2020
(73) Proprietor: Withings, 92130 Issy les Moulineaux (FR)
(72) Inventor: ZHANG, Xianzhi, 75006 PARIS (FR); WANG, Jean-Louis, 92800 PUTEAUX (FR); VISSAC, Virginie, 78370 PLAISIR (FR); CUNIASSE, Pierre-Antoine, 92220 BAGNEUX (FR); TUCOULAT, Benoît, 75010 PARIS (FR); ALSBERGHE-PATEL, Mélanie, 75014 PARIS (FR)
(74) Representative: Plasseraud IP

(56) References cited:
- EP-A1- 0 705 563
- EP-A1- 3 375 358
- WO-A1-2018/094736
- US-A1- 2012 265 240

## Description

### FIELD OF THE DISCLOSURE

The present disclosure relates to devices for analysing cardiovascular parameters of an individual. More particularly, it relates to a multifunction cuff-type monitoring device for monitoring cardiovascular parameters of such individual.

### BACKGROUND OF THE DISCLOSURE

Such device is configured to collect cardiovascular parameters like blood pressure and at least one additional signal, in particular an electrical signal representative of an electrocardiogram or an acoustic signal representative of a phonocardiogram. More specifically, there is provided an inflatable bladder to pressurize the arm of the patient. Further the monitoring device comprises on the one hand a cuff assembly configured, in use, to surround an arm of the individual, and on the other hand a control unit housing which forms together with the cuff assembly a single unit. Since the control unit housing is somewhat tubular, the link member between the cuff assembly and the control unit housing should be simple and mechanically robust. But it is necessary to provide various connections (electrical, pneumatic) between the cuff assembly and the control unit housing, which is somehow contradictory with the goal previously set out. The inventors have endeavored to find a smart solution to respond to various combined constraints.

EP3375358A1 discloses a device for collecting cardiovascular data. US2012/265240A1 discloses a system for performing remote ischemic conditioning. WO2018/094736A1 discloses a unitary wearable electronic sphygmomanometer cuff.

### SUMMARY OF THE DISCLOSURE

According to one aspect of the present invention it is disclosed a cuff-type monitoring device, for collecting cardiovascular data relating to an individual user, comprising :
a control unit housing including at least a pump, a cuff assembly including a resilient cuff holder and configured, in use, to surround an upper limb of the individual, the cuff assembly having an internal wall, the cuff assembly further comprising a first contact electrode arranged on the internal wall; and
an interface member arranged at the control unit housing, provided as a separate part, wherein there is provided at least a pneumatic coupling and at least an electrical coupling through the interface member, for coupling the cuff assembly to the control unit housing, wherein the resilient cuff holder comprises two retaining members, the interface member comprising two complementary retaining members, configured to be snap-fit with the retaining members; wherein the interface member exhibits a first face oriented towards control unit housing and a second face oriented towards the cuff assembly, wherein the first face is concave and is configured to follow a cylindrical body of the control unit housing;
wherein there is provided an ECG electrode disposed around at least a part of the control unit housing and the interface member is configured to securely fix the ECG electrode on the control unit housing;
and wherein the electrical coupling comprises a connection wire coupling the contact electrode with the control unit housing.

Thanks to these dispositions, the assembly of such device is very simple. The assembled device is aesthetic, since no pneumatic coupling and no electrical coupling is visible from the outside of the device.

In the present document, the geometric reference may be defined as follows for the interface member : the assembling direction is denoted **R,** the elongation axis of the interface member is denoted **X,** the transverse direction of the interface member is denoted **T.**

Besides, one or more of the following features can be used optionally, taken alone or in combination.

According to an option, the interface member is a separate part interposed between the control unit housing and the cuff assembly for securing them together. The interface member can be manufactured from a material different than the control unit housing. Each of these two parts can have differentiated constraints and mechanical requirements. Each of these two parts are manufactured and prepared independently from one another.

According to an option, the interface member is screwed to the control unit housing. Screwing is well mastered and forms a reliable mechanical solution for fastening function.

The interface member may comprise a frustoconical thru bore for each screw. Each of such thru bore is designed to receive a countersunk head screw that will not protrude from a face of the interface member. This assembly is therefore flush and is thus not aggressive for the cuff assembly. There may be provided 2 or 4 screws in the attachment.

According to a particular option, the interface member exhibits a symmetry with regard to plane X,R. Thereby the interface member is rather simple to manufacture

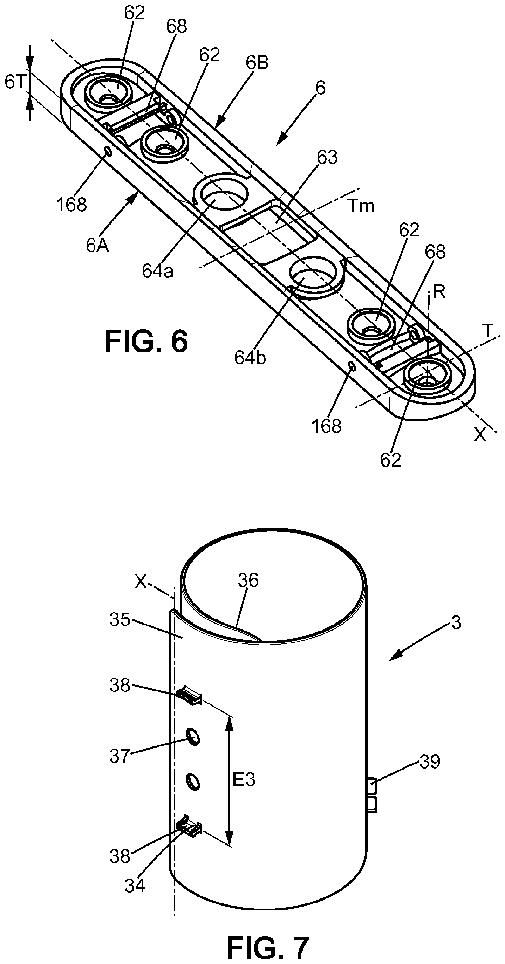

According to a particular option, the interface member exhibits a symmetry with regard to a median plane Tm (cf Fig 6). It prevents the risk of mistake when assembling the interface member.

According to a particular option, the interface member exhibits a double symmetry with regard to plane Tm and with regard to plane X,R. Manufacturing of such item is therefore simplified.

According to a particular option, the cuff holder can be a monolithic resilient part.

According to an option, the retaining members may be formed as hooks. Such hooks protrude from the cuff holder baseline layer. There may be provided a large footprint for these hooks, namely a foot/shoe larger than the hook arm profile itself, thereby providing a strong implant on the cuff holder sheet; large disassembly torque can thus be withstood.

According to an option, there is provided two hooks spaced from one another of a distance (E3) at least 5 cm or at least 50% of the height of the cuff assembly. Here the height is taken along the elongation axis of the interface member X. The assembly can thus withstand large disassembly torque.

According to an option, there is provided interlock members (138) received in holes along a transverse direction T. The interlock members prevent disassembly of complementary retaining members versus retaining members.

According to an option, the interface member comprises transverse holes (168) for receiving the interlock members (138), said transverse holes having preferably a diameter less than 2mm. Advantageously, there is no screw visible outside the device; the interface member can be a thin, elegant part.

According to an option, the interlock elements (138) can be formed as interlock pins. According to another option, the interlock elements (138) can be formed as interlock thin screws, for example, rod-like screw with sotted head.

According to the invention, the interface member (6) exhibits a first face (6A) oriented towards the control unit housing (7) and a second face (6B) oriented towards the cuff assembly, wherein the first face is concave and is configured to follow the cylindrical body of the control unit housing. Therefore, the contact area between the interface member and the control unit housing is maximized and the mechanical attachment is therefore made stronger.

According to an option, the second face is substantially flat. The contact of the cuff assembly is smooth and not aggressive.

According to an option, both faces may also be concave, in particular when the interface member (6) may have a large transverse dimension, the second face (6B) may exhibit a slight concavity to follow the concavity of the cuff holder.

According to an option, the cuff holder is a resilient plastic part with retaining members or hooks integrally formed therein. Such part is easy to manufacture and assembly is simplified.

According to an option, the interface member is an elongated flat part, plastic part, preferably with a thickness (6T) along assembling direction R less than 6 mm. Thereby, with such a thin interface member, the cuff-type monitoring device exhibit an aesthetic appearance and is a compact solution. The length of the interface member along the elongation axis is preferably at least 10 cm.

According to an option, there is provided in a middle portion of the interface member at least a through hole for electrical wire connection/coupling, and a least one through hole for pneumatic fluid piping /coupling. Not only one multi-wire connection is possible but also two pneumatic fluid connections are provided internally to the interface member, i.e. through holes arranged in the interface member.

According to the invention, there is provided an ECG electrode (32) disposed around at least a part of the control unit housing (7) and the interface member (6) is configured to securely fix the ECG electrode on the control unit housing. Advantageously, the interface member has an additional electrical function.

The present invention is also directed to an **assembly method** for assembling a cuff-type monitoring device, for collecting cardiovascular data relating to an individual as defined in claim 10.

According to an option, the method may further comprise :
- disassembling the cuff assembly from the interface member, by lifting the intermediate portion of the cuff assembly from the interface member.

According to an option, the method may further comprise :
- electrically connecting a plug arranged with the cuff assembly into a socket arranged in the control unit housing. Prior to assembling the cuff assembly to the interface member.

### BRIEF DESCRIPTION OF THE DRAWINGS

Other features and advantages of the disclosure appear from the following detailed description of one of its embodiments, given by way of non-limiting example, and with reference to the accompanying drawings, in which:
- Figure 1 illustrates a general overview of a monitoring device according to the present disclosure in a use configuration,
- Figure 2 shows a diagrammatic sectional view of the device in place on the left arm of the user, and adjacent to the chest, for the generically defined embodiment,
- Figure 3 illustrates a picture of the device when not in use,
- Figure 4 shows partially the monitoring device illustrating in particular a control unit housing and a resilient cuff holder attached via an interface member,
- Figure 5 shows an exploded view of the monitoring device,
- Figure 6 is a perspective view of an example of the interface member,
- Figure 7 is a perspective view of an example of resilient cuff holder,

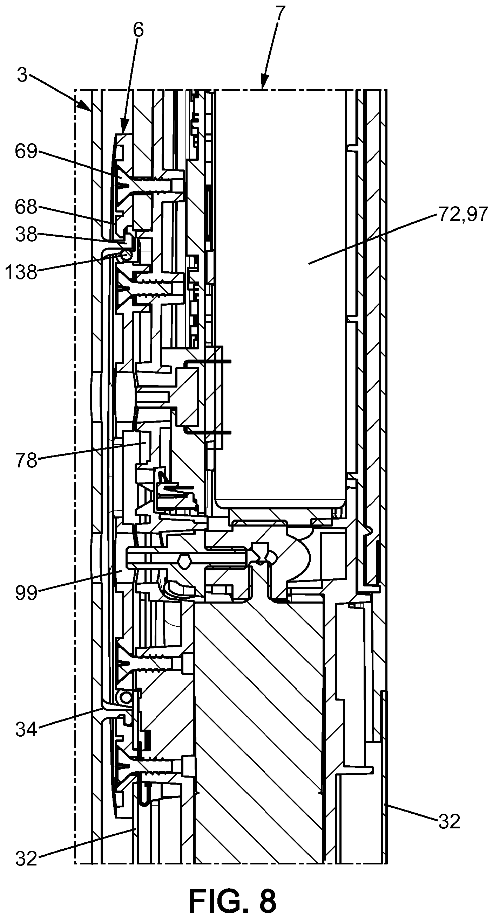

- Figure 8 is an enlarged view of the interface area, also illustrating the interlocking function,
- Figure 9 shows a sectional view of the interface member and associated elements,
- Figure 10 shows a functional block diagram.

### DETAILED DESCRIPTION OF THE DISCLOSURE

In the figures, the same references denote identical or similar elements. For clarity purposes, some parts are represented intentionally not at scale with regard to other parts. Also, some parts of timing charts can be represented intentionally not at scale.

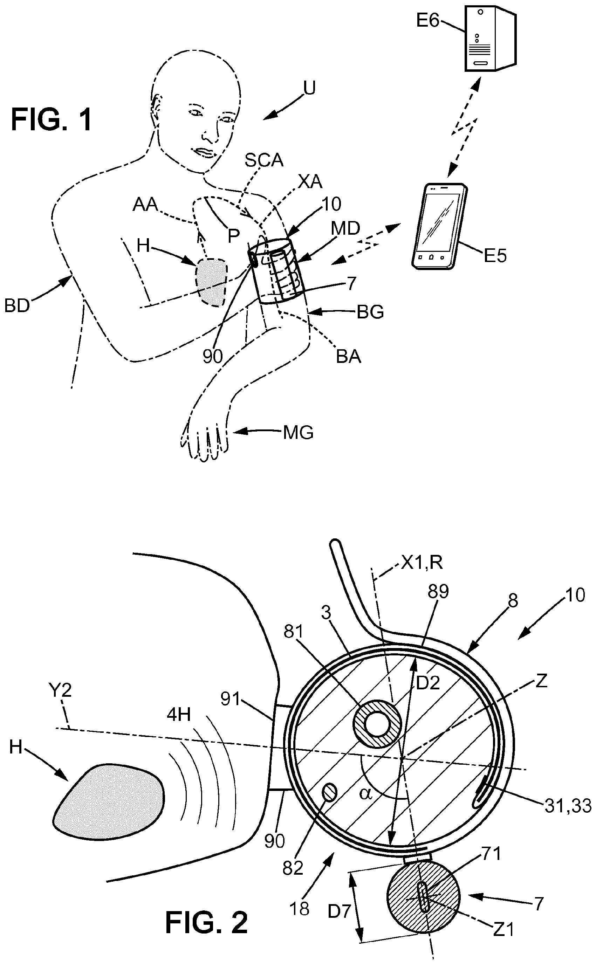

Figure 1 shows an individual (also 'user') U in a configuration where he/she is using a monitoring device according to the present disclosure. The device (otherwise called "apparatus") looks like a known brachial blood pressure sensing device (commonly named Blood Pressure Monitor i.e. in short "BP Monitor"), but the device exhibits extended functionalities as will be apparent below, so that the device can be called 'upgraded BP Monitor' .

The user **U** in question has, among other organs and limbs in his/her anatomy : a left arm **BG,** a right arm **BD,** a heart **H,** a left hand **MG** a right hand **MD.**

Further the user in question has an aortic artery ('aortic arch') denoted **AA,** a subclavian artery **SCA,** an axillary artery **XA,** a brachial artery **BA,** belonging generally to the cardiovascular system of the user. Therefore a blood path of interest noted **P** is defined as the fluid conduit from the heart **H** to a reference point at the brachial artery **BA.**

The device **10** has a wireless communication capability to exchange data with a mobile entity like a smartphone **E5** (more generally a mobile device belonging to the user U like a tablet, a laptop....). Such smartphone **E5** may in turn exchange data with a remote entity like an Internet server **E6** (more generally any resource available somewhere in Internet, not excluding a so-called "cloud" resource).

The monitoring device **10** has either a small display or no display at all, since the user interface capability provided by the smartphone **E5** is fully relevant to support displays relating to the use and extended functionalities of the device.

The monitoring device **10** is intended to be used at a home environment, for healthy people as well as people suffering from some disease. It may be used in a medical environment but is particularly suitable to be used by non-medical personnel, i.e. the user under measurement him/herself.

The monitoring device **10** comprises a cuff assembly **18** wrapped around the arm **BG,** a control unit housing 7. The cuff assembly **18** will be described in more details later. The cuff assembly **18** is coupled to the control unit housing 7 via an interface member **6** whose purpose and details will be given later.

The rest of the time the monitoring device is stowed, notably in a folded configuration as will be seen later.

### Cuff assembly

As illustrated on Figure 1, the device comprises a cuff assembly ('armband' can also be used) wrapped around the arm i.e. the part of the upper limb comprised between the shoulder and the elbow.

In another configuration or variant, the device can be used elsewhere, at the forearm for example, or at the wrist. Generally speaking, the cuff assembly **18** is configured, in use, to surround an upper limb of the user.

As illustrated on Figure 1, the device is installed on the left arm of the user. However it is not excluded to use the device elsewhere, at the right upper limb for example.

As illustrated on Figure 2, the left arm of the user includes a bone named humerus denoted **81,** muscles (not especially shown), and the above mentioned brachial artery denoted **82.** The humerus extends along an axis denoted **Z.** The cuff assembly **18,** when wrapped around the arm BG, has a general cylindrical shape with a reference axis substantially coinciding with arm axis **Z.**

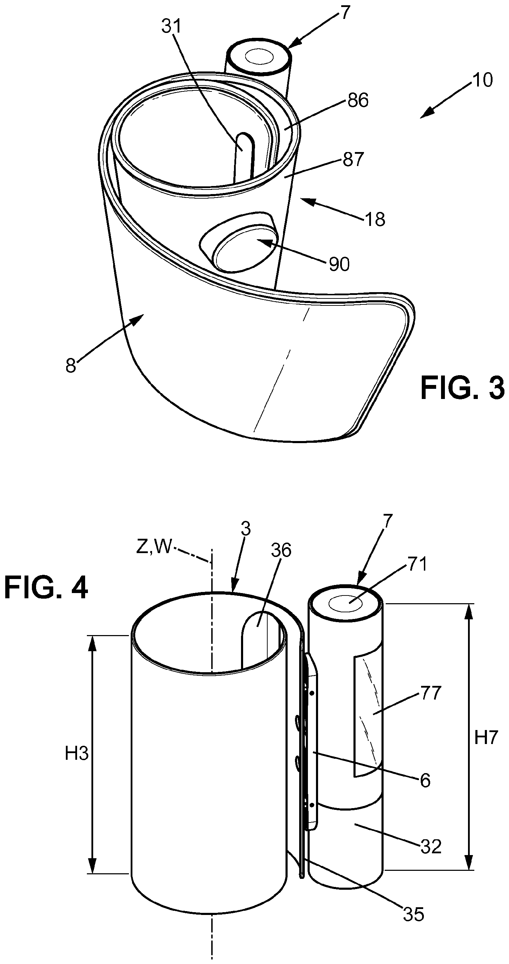

As shown at figures 3 to 5, the cuff assembly **18** comprises, from an innermost item to an outermost item, at least the following components :
- an inner layer, denoted **1,**
- an inflatable bladder, denoted **2,**
- an cuff holder, denoted **3,**
- an intermediate sheet, denoted **4,**
- an outer band, denoted **5.**

Regarding overall dimensions of the cuff assembly **18,** at flat developed configuration, the length **L1** of the cuff assembly along the longitudinal direction L can be comprised between 20 cm and 40 cm. The height **H1** along the transverse axis W direction can be comprised between 10 cm and 20 cm.

The inner layer 1 is a thin fabric layer. The inner layer **1** may be made in lycra. Lycra turns out to be a comfortable material, not prone to collect dirt and/or sweat. The inner layer 1 is optional in the frame of the present invention, in particular when the outer wall directed inwardly of the bladder 2 is texturized (likewise textured).

The inflatable bladder **2** is made from two sheets **2A,2B** of plastic material (for instance PVC, TPU, PA, etc) arranged one atop another in a main plane and are welded together at their peripheral border.

The inflatable bladder **2** has two fluid connection ports denoted **99,199.** One port is for inflating, deflating, whereas the other one is dedicated to a measuring channel, so that the pressure measured is not perturbed by the unsteady flow of the pump during inflation. In other configurations, there may be only one port for both functions.

The connection ports denoted **99,199** extend perpendicularly to the main plane, along the thickness direction **Y** of the cuff assembly and along the assembling direction R of interface member **6.** Air volume at nominal inflated state can be comprise between 0,05 liter and 0,2 liter.

The cuff holder **3** forms a structural resilient armature of the cuff assembly. The cuff holder is a resilient plastic sheet, with a first longitudinal end **35** attached to the interface member 6 and a second end **36** having a tapered end portion, i.e. with a decreasing thickness to the edge; the first longitudinal end **35** is called proximal and the second end **36** is called distal.

The dimensions of the cuff holder at flat configuration are slightly less than the corresponding dimensions of the bladder.

The thickness of the cuff holder can be comprised between 0,5 mm and 3 mm.

The cuff holder can be made out of polypropylene.

The second end 36 has advantageously a tapered end portion, i.e. a decreasing thickness down to the edge.

The resilient cuff holder **3** comprises two retaining members **38** at the first longitudinal end **35.** In the shown example, the retaining members are formed as hooks, arranged in symmetry with regard to a median plane **Tm** (cf Fig 6). The hooks are spaced from one another by a distance **E3. E3** is at least 5 cm, or at least 50% of the height H1 of the cuff assembly.

The hook is arranged at an end of a hook arm. In the shown example, there is provided a large footprint for these hook arms, with a rounded shape **34,** also named 'fillet'. The foot/shoe is therefore larger than the hook arm profile itself. It provides a strong implant on the cuff holder sheet.

The resilient cuff holder **3** comprises two through-holes **37,** configured to let a suitable passage for the fluid ports **99,199** of the inflatable bladder.

The resilient cuff holder 3 comprises protrusions **39** providing a socket for the attachment of an acoustic sensor **90** and its mechanical securing thereto.

The intermediate sheet **4** is made in an inextensible, i.e. non-stretchable fabric.

The intermediate sheet **4** may be made in a nylon fabric.

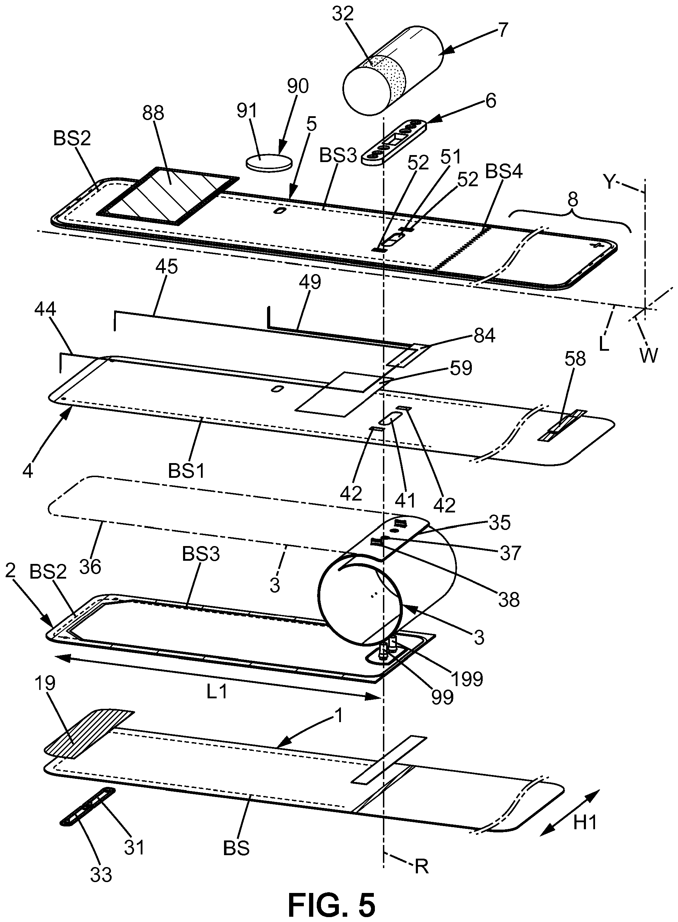

Besides, for the purpose of letting a passage for the one or two fluid ports **99,199** of the inflatable bladder, there is provided a first opening **41** in the intermediate sheet **4** as illustrated at figure 5.

Further, the cuff assembly **18** is fixed to the control unit housing, and there are provided second openings **42** in the intermediate sheet 4 to let the passage for at least two hooks of the cuff holder, said hooks being configured to be retained in the control unit housing 7 or an attachment member 6 thereof.

The outer band **5** constitutes the external layer. The outer band **5** can comprise one or more foamed areas. The outer band **5** can comprise fixing means (hooks and loops [i.e. Velcro^{™}] or the like). There may be provided a magnet **58** and a ferrite or metal pad **59** for rollover stowed configuration. The magnet **58** or ferrite **59** can be arranged with the intermediate sheet **4** or with the outer band 5.

The outer band **5** can comprise an extension beyond the cuff assembly forming part or all of an attachment band **8** discussed later.

Besides, for the purpose of letting a passage for the one or two fluid ports **99,199** of the inflatable bladder, there is provided a first opening **51** in the outer band 5 as illustrated at figure 5.

Further, the cuff assembly **18** is fixed to the control unit housing, and there are provided second openings **52** in the outer band 5 to let the passage for at least two hooks of the cuff holder, said hooks being configured to be retained in the control unit housing 7 or an attachment member 6 thereof.

### Border joint or seam

There is provided a border joint at least on three sides on a peripheral border of the inflatable bladder, the border joint joining and securing together the inner layer 1 (optionally), the inflatable bladder 2, the intermediate sheet 4, and the outer band 5.

In the shown example, the border joint is a border seam. There are provided a first border seam **BS1,** a second border seam **BS2,** and a third border seam **BS3,** collectively denoted **BS.**

There may be provided in fourth border seam **BS4** which is made after cuff holder 3 has been inserted inside the cuff assembly between the bladder **2** and the intermediate sheet **4.**

According to an alternative solution, instead of making a seam, it is possible to use a technique of ultrasonic or thermal welding.

### Control unit housing

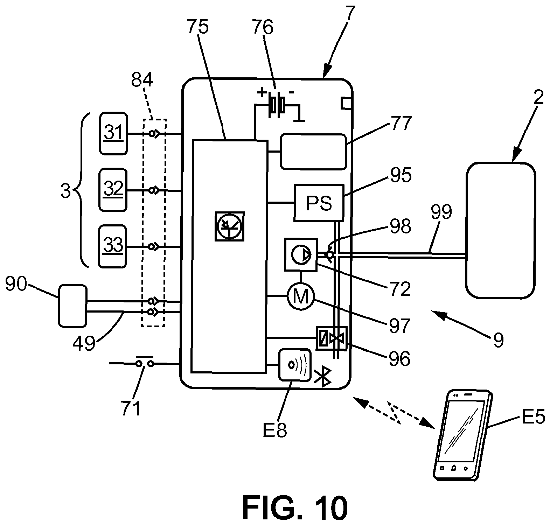

As apparent from figure 10, the control unit housing **7** comprises a pneumatic unit **9.**

The control unit housing **7** comprises an electronic controller **75** configured to control the pneumatic unit and to determine at least the arterial pressure of the user,

The control unit housing **7** comprises an On/Off switch **71** and a display **77.**

The pneumatic unit **9** comprises the already mentioned pump **72** driven by an electrical motor **97,** a discharge valve **96,** a check valve **98,** and a pressure sensor **95.**

The control unit housing **7** exhibits generally a cylindrical shape. The diameter **D7** of the control unit housing **7** is preferably less than 40mm, preferably around 35 mm. The proposed configuration turns out to be a compact solution which accommodates all the necessary components for the measurement, including a battery **76** for autonomous use.

### Interface member

As already mentioned, with reference to Fig 6, the assembling direction is denoted **R,** the elongation axis of the interface member is denoted **X,** the transverse direction of the interface member is denoted **T. Tm** designates a median plan parallel to R and T.

According to the illustrated example, the interface member **6** is a separate part interposed between the control unit housing and the cuff assembly for securing them together.

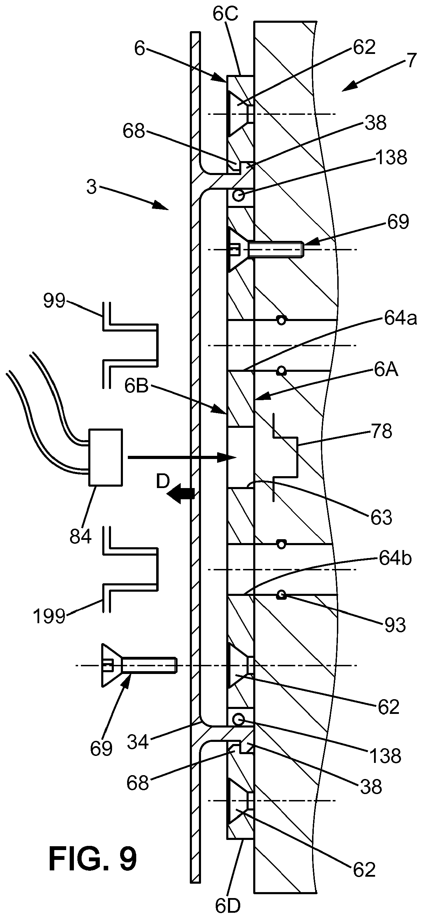

As illustrated at figures 6 and 9, the interface member 6 is screwed to the control unit housing. More precisely, there are provided through holes **62** in the interface member.

Screws **69** are provided to fix the interface member to the control unit housing.

In the shown example, the through holes **62** are frustoconical thru bore, and the proposed screws are of the type countersunk head screw.

There is provided in a middle portion of the interface member at least a through hole **63** for electrical wire connection/coupling, two through holes **64a,64b** for pneumatic fluid piping /coupling.

There is provided threaded holes in the body of the control unit housing 7 for receiving the screws **69.** There may be provided 2 or 4 screws in the attachment (4 illustrated).

The interface member **6** comprises two complementary retaining members **68,** configured to be snap-fit with the retaining members 38. Here the complementary retaining members are in the form of shoulders or elbows, i.e. stop abutments preventing retaining members from moving away from the control unit housing.

Advantageously, the free extremities of the retaining members 38 are directed towards the edges of the cuff assembly, i.e. away from each other. Thanks to the resilience of the cuff-holder 3, this system makes it easy to disassemble the cuff assembly from the control unit housing 7 by applying an inward pressure (arrow **D** at fig 9) on the cuff assembly (in the R-direction) near the intermediate portion of the interface member **6.** Assembly and disassembly require no additional tool or machinery, are very fast, and can thus be repeated at will, which is very convenient for testing and changing parts of the device.

In one embodiment, the interface member 6 comprises transverse holes **168** arranged in the transverse direction (T) for receiving the interlock pins **138.** The interlock pins prevent disassembly of complementary retaining members **68** versus retaining members **38.** Said interlock pins 138 and transverse holes 168 have preferably a diameter less than 2mm, therefore, this is nearly hidden and difficult to tamper.

Instead of interlock pins, any other mechanical parts having an interlocking function can be used like thin rod-like screw with slotted head. Therefore, the clause *"interlock element"* is generically used.

We note that interlock elements 138 can easily be removed, making possible and easy to disassemble the cuff-assembly from the control unit housing 7. We note here that the hooks 68 are oriented away from each other.

The interface member 6 exhibits a first face **6A** oriented towards the control unit housing 7 and a second face **6B** oriented towards the cuff assembly.

The first face **6A** is concave and is configured to follow the cylindrical body of the control unit housing. The second face 6B is substantially flat or exhibits a slight concavity.

The longitudinal ends of the interface member denoted respectively 6C and 6D. The distance between the two ends 6C,6D is at least 10 cm or two-thirds of the height H1 of the cuff-holder.

There is provided a connector **84** attached to connection wires discussed later. In one example, the connector is a flat flex PCB-type connector. The connector is inserted through the central through hole **63,** and is plugged onto a corresponding counterpart electrical socket **78** provided in the control unit housing.

Regarding the pneumatic connections, there may be provided O-rings **93** in the control unit housing or likewise complementary tubes, such that the connection ports 99,199 are received in an airtight manner into pneumatic components of the control unit housing.

It is also apparent from figure 9 that disassembling the cuff assembly from the interface member is possible provided that the interlock members are not yet inserted. More precisely, if a pressing effort is exerted along the arrow **D,** the hooks tend to move a bit toward each other therefore enabling disassembly of the hooks from the retaining shoulders.

### Attachment band

There is provided an attachment band **8.** The attachment band **8** extends from the cuff assembly along a longitudinal direction **L** configured to momentarily secure the attachment band to the outer band of the cuff assembly during a measurement cycle.

The attachment band **8** is flexible and comprises fixing means for securing that number to the external wall **87** of the cuff assembly **18** at least during the blood pressure measurement. The proper position is chosen by the user according the size of his/her arm.

In one configuration, the fixing means may comprise loop and hook pads, a loop pad **88** at one location and a hook pad **89** at another counterpart location, such that adjustment and securing of various encompassed circumferences (π D2) of user's arm is made available. This allows convenient attachment whatever the size of the arm.

### Sensors (Stetho and ECG)

The device **10** may comprise additionally an acoustic sensor denoted **90.**

The cuff assembly **18** has an internal wall denoted **86** intended to contact the arm's skin (or light clothing) and to press against the arm. The armband has an external wall denoted **87** on the opposite side of the band with regard to the internal wall **86.**

In use configuration, the acoustic sensor **90** is located against the chest, i.e. the left side of chest. Sound waves **4H** emitted by the heart are sensed by a sensitive portion **91** of the acoustic sensor **90** the sensitive portion **91** bearing on the left-side chest, i.e. adjacent to the chest. Acoustic waves **4H** are converted into electrical signals as known per se thus not detailed here. It should be noted that acoustic waves **4H** can be sensed without trouble through a light clothing, an underwear or the like.

According to the invention, the device is further equipped with an ECG function, i.e. ElectroCardioGraphic function.

For this purpose there are provided three contact electrodes **31, 32, 33,** the three of them integrated in the device, without the need to have linking wires like in most prior art devices.

The first electrode **31** is arranged on the internal wall **86** of the band and has a sensitive face oriented toward the skin of the arm. The third electrode **33** is also arranged on the internal wall **86** and has also a sensitive face oriented toward the skin of the arm.

Each of the electrodes is formed as a thin pad of a surface comprised between 2 cm² and 10 cm²; a surface between 5 cm² and 7cm² can be chosen; the shape of the thin pad may be somewhat curved to follow the standard curvature of the skin of the arm.

The first and third electrodes **31,33** are arranged at a cuff assembly distal end (second end **36**) opposed to the interface member for example one aside the other transversally (along **W**). Alternatively, first and third contact electrodes **31, 33** can be arranged differently, for example one aside the other along **L.** In the shown example, there is provided a back pad **19** to support the contact electrodes **31, 33.** The Lycra inner layer 1 is sandwiched at this place between the back pad **19** and the contact electrodes **31, 33.**

Whenever the cuff assembly is pressurized, first and third contact electrodes **31, 33** are firmly pressed against the skin of the arm, thereby ensuring a fairly good contact with a small electrical contact resistance. It should be noted that no gel is required at the contact electrode contrary to prior art habits. Contact electrodes are to be placed against the bare skin; however, thanks to the pressure, it is not excluded to have a light underwear cloth between the skin and the electrodes.

The contact electrodes can be made of stainless steel, silver, or other coated materials (coated with silver, chromium, gold, titanium or platinum), not excluding materials coated by physical vapor deposition technique (known as PVD techniques). The contact electrodes can also be made of a conducting wire (stainless steel, silver, etc) sewed to the inner layer.

It is to be noted that two electrodes might be sufficient, therefore the third electrode **33** is considered optional.

Regarding the second electrode **32,** it is arranged around the external surface of the control unit assembly as best seen at figure 4. A conductive material forms a coating of at least a part of the control unit housing. A metallic coating material (silver, titanium, chromium), are deposited by physical vapor deposition technique (known as PVD techniques). It can also be an empty cylinder made out of the conductive material.

In one example, the second electrode covers the lower third of the cylinder, for example all around the accessible circumference by the fingers of the user (see Fig 1). Therefore, it is easy for the user to grab/seize the second electrode with a good electrical contact. According to one example, the second electrode lies over the 20% lower part of the control assembly unit; in other implementation, its height can be bigger, like 30% or 40%.

The device further comprises connection wires to provide electrically coupling between sensors and the control unit housing **7.** Connection wires **44,45** electrically couple the contact electrodes with the control unit housing.

Connection wires **49** are provided to electrically couple the acoustic sensor with the control unit housing. The connection wires are integrated into the cuff assembly and they are protected therein and not visible from the outside. As depicted at figure 5, since the connection wires are arranged at an outer zone of the bladder, preferably between the intermediate sheet (4), and the outer band (5), they do not hinder inflation and measurement.

The wires 44,45,49 are electrically coupled to the above mentioned connector **84.**

### Overall system

The control unit housing **7,** the attachment band **8** and the cuff assembly have substantially the same height **(H1,H3,H7)** along the transverse axis **W** (coincides with elongation axis **X** for the interface member). This provides an aesthetic assembly and eases rollup configuration. This configuration allows optimization of the occupied volume.

As shown at figure 2, the control unit housing **7** and the acoustic sensor 90 are arranged at an angle **α** comprised between 90° and 140° with regard to the cylindrical configuration of the cuff assembly, as defined in a use configuration on a 30cm reference arm circumference.

As shown at Figure 10, there is provided a wireless coupling **E8** with smartphone **E5.**

It is to be noted that the switch **71** can be physical switch, or tap switch or a tactile area.

It shall be understood that the use of interlock member(s) is not compulsory. In one embodiment, there may be provided strong snap-fit connection retaining members and complementary retaining members. There can be provided two or more hooks and complementary shoulders or stop abutments. The various hooks can exhibit different orientations so that mechanical torques along different orientation can be with withstood without disassembly.

## Claims

1. A cuff-type monitoring device (10), for collecting cardiovascular data relating to an individual user, comprising :
- a control unit housing (7) including at least a pump,
- a cuff assembly (18) including a resilient cuff holder (3) and configured, in use, to surround an upper limb of the individual, the cuff assembly having an internal wall (86), the cuff assembly further comprising a first contact electrode (31) arranged on the internal wall (86); and
- an interface member (6) arranged at the control unit housing, provided as a separate part, wherein there is provided at least a pneumatic coupling and at least an electrical coupling through the interface member, for coupling the cuff assembly to the control unit housing, wherein the resilient cuff holder comprises two retaining members (38),
the interface member (6) comprising two complementary retaining members (68), configured to be snap-fit with the retaining members (38);
wherein the interface member (6) exhibits a first face (6A) oriented towards control unit housing (7) and a second face (6B) oriented towards the cuff assembly, wherein the first face is concave and is configured to follow a cylindrical body of the control unit housing;
wherein there is provided an ECG electrode (32) disposed around at least a part of the control unit housing (7) and the interface member (6) is configured to securely fix the ECG electrode on the control unit housing.; and
wherein the electrical coupling comprises a connection wire coupling the contact electrode with the control unit housing.

2. The device according to claim 1, wherein the interface member (6) is a separate part interposed between the control unit housing and the cuff assembly for securing them together.

3. The device according to claim 2, wherein the interface member is screwed to the control unit housing.

4. The device according to any of the claims 1 to 3, wherein the retaining members are formed as hooks (38).

5. The device according to claim 4, wherein there is provided two hooks spaced from one another of a distance (E3) at least 5 cm, or at least 50% of the height of the cuff assembly.

6. The device according to any of the claims 1 to 5, wherein there is provided interlock elements / pins (138) in holes along the transverse direction (T), and the interface member (6) comprises transverse holes (168) arranged in the transverse direction (T) for receiving the interlock pins (138).

7. The device according to any of the claims 1 to 6, wherein the cuff holder (3) is a resilient plastic part with retaining members or hooks integrally formed therein.

8. The device according to any of the claims 1 to 7, wherein the interface member is an elongated flat part, plastic part, preferably with a thickness (6T) along assembling direction (R) less than 6 mm.

9. The device according to any of the claims 1 to 8, wherein there is provided in a middle portion of the interface member at least a through hole for electrical wire connection and at least one through holes (64a,64b) for pneumatic fluid piping.

10. An assembly method for assembling a cuff-type monitoring device according to claim 6, for collecting cardiovascular data relating to an individual user, the method comprising:
- Providing the control unit housing (7) including at least a pump,
- Providing the cuff assembly (10) including the resilient cuff holder (3) and
- Providing the interface member (6)
- Fixing the interface member to the control unit housing, by screwing
- Assembling the cuff assembly to the interface member, by inserting the two retaining members into the two complementary retaining members,
- Securing the cuff assembly to the interface member by inserting the interlock elements (138).

11. The assembly method according to claim 10, further comprising :
- electrically connecting a plug arranged with the cuff assembly into a socket arranged in the control unit housing, prior to assembling the cuff assembly to the interface member.

12. The assembly method according to claim 10, wherein the interface member is fixed to the control unit housing by screwing.

## Patentansprüche

1. Manschettenartige Überwachungsvorrichtung (10) zum Sammeln von kardiovaskulären Daten, die einen einzelnen Benutzer betreffen, die aufweist:
- ein Steuereinheitsgehäuse (7), das mindestens eine Pumpe enthält,
- eine Manschettenanordnung (18), die einen elastischen Manschettenhalter (3) aufweist und konfiguriert ist, im Gebrauch eine obere Extremität der Person zu umgeben, wobei die Manschettenanordnung eine Innenwand (86) aufweist und die Manschettenanordnung ferner eine erste Kontaktelektrode (31) aufweist, die an der Innenwand (86) angeordnet ist; und
- ein am Steuereinheitsgehäuse angeordnetes Schnittstellenelement (6), das als getrenntes Teil vorgesehen ist, wobei mindestens eine pneumatische Kupplung und mindestens eine elektrische Kupplung durch das Schnittstellenelement vorgesehen ist, um die Manschettenanordnung mit dem Steuereinheitsgehäuse zu koppeln, wobei der elastische Manschettenhalter zwei Halteelemente (38) aufweist,
wobei das Schnittstellenelement (6) zwei komplementäre Halteelemente (68) aufweist, die konfiguriert sind, in die Halteelemente (38) einzuschnappen;
wobei das Schnittstellenelement (6) eine erste Fläche (6A), die zum Gehäuse (7) der Steuereinheit ausgerichtet ist, und eine zweite Fläche (6B) aufweist, die zur Manschettenanordnung ausgerichtet ist, wobei die erste Fläche konkav ist und konfiguriert ist, einem zylindrischen Körper des Gehäuses der Steuereinheit zu folgen; wobei eine EKG-Elektrode (32) vorgesehen ist, die um mindestens einen Teil des Steuereinheitsgehäuses (7) angeordnet ist, und das Schnittstellenelement (6) konfiguriert ist, die EKG-Elektrode sicher am Steuereinheitsgehäuse zu befestigen; und
wobei die elektrische Kopplung einen Verbindungsdraht aufweist, der die Kontaktelektrode mit dem Steuereinheitsgehäuse koppelt.

2. Vorrichtung nach Anspruch 1, wobei das Schnittstellenelement (6) ein separates Teil ist, das zwischen dem Steuereinheitsgehäuse und der Manschettenanordnung angeordnet ist, um sie aneinander zu sichern.

3. Vorrichtung nach Anspruch 2, bei der das Schnittstellenelement an das Steuereinheitsgehäuse geschraubt ist.

4. Vorrichtung nach einem der Ansprüche 1 bis 3, wobei die Halteelemente als Haken (38) ausgebildet sind.

5. Vorrichtung nach Anspruch 4, wobei zwei Haken vorgesehen sind, die voneinander mit einem Abstand (E3) von mindestens 5 cm oder mindestens 50% der Höhe der Manschettenanordnung beabstandet sind.

6. Vorrichtung nach einem der Ansprüche 1 bis 5, wobei Verriegelungselemente/Stifte (138) in Löchern entlang der Querrichtung (T) vorgesehen sind und das Schnittstellenelement (6) Querlöcher (168) aufweist, die in der Querrichtung (T) zur Aufnahme der Verriegelungsstifte (138) angeordnet sind.

7. Vorrichtung nach einem der Ansprüche 1 bis 6, wobei der Manschettenhalter (3) ein elastisches Kunststoffteil mit Halteelementen oder Haken ist, die darin integral ausgebildet sind.

8. Vorrichtung nach einem der Ansprüche 1 bis 7, wobei das Schnittstellenelement ein längliches, flaches Teil, Kunststoffteil ist, vorzugsweise mit einer Dicke (6T) entlang der Montagerichtung (R) von weniger als 6 mm.

9. Vorrichtung nach einem der Ansprüche 1 bis 8, wobei in einem mittleren Abschnitt des Schnittstellenelements mindestens ein Durchgangsloch für eine elektrische Drahtverbindung und mindestens ein Durchgangsloch (64a, 64b) für eine pneumatische Fluidleitung vorgesehen ist.

10. Montageverfahren zum Montieren einer manschettenartigen Überwachungsvorrichtung nach Anspruch 6 zum Sammeln von kardiovaskulären Daten, die einen einzelnen Benutzer betreffen, wobei das Verfahren aufweist:
- Bereitstellen des Steuereinheitsgehäuses (7), das mindestens eine Pumpe enthält,
- Bereitstellen der Manschettenanordnung (10) die einen elastischen Manschettenhalter (3) aufweist, und
- Bereitstellen des Schnittstellenelements (6)
- Befestigen des Schnittstellenteils am Steuereinheitsgehäuse durch Verschrauben
- Montieren der Manschettenanordnung am Schnittstellenelement, durch Einsetzen der beiden Halteelemente in die beiden komplementären Halteelemente,
- Sichern der Manschettenanordnung am Schnittstellenelement durch Einsetzen der Verriegelungselemente (138).

11. Montageverfahren nach Anspruch 10, das ferner aufweist:
- elektrisches Verbinden eines Steckers, der mit der Manschettenanordnung angeordnet ist, mit einer Buchse, die im Steuereinheitsgehäuse angeordnet ist, vor dem Montieren der Manschettenanordnung am Schnittstellenelement.

12. Montageverfahren nach Anspruch 10, wobei das Schnittstellenelement durch Verschrauben am Steuereinheitsgehäuse befestigt wird.

## Revendications

1. Dispositif de surveillance de type à brassard (10), pour collecter des données cardiovasculaires concernant un utilisateur individuel, comprenant :
- un logement d'unité de commande (7) incluant au moins une pompe,
- un ensemble brassard (18) incluant une armature de brassard élastique (3) et configuré, durant l'utilisation, pour entourer un membre supérieur de l'individu, l'ensemble brassard ayant une paroi interne (86), l'ensemble brassard comprenant en outre une première électrode de contact (31) agencée sur la paroi interne (86) ; et
- un élément interface (6) agencé au niveau du logement d'unité de commande, fourni comme une pièce séparée, dans lequel sont fournis au moins un raccord pneumatique et au moins un raccord électrique à travers l'élément interface, pour raccorder l'ensemble brassard au logement d'unité de commande, dans lequel l'armature de brassard élastique comprend deux éléments de retenue (38),
l'élément interface (6) comprenant deux éléments de retenue complémentaires (68), configurés pour être encliquetés avec les éléments de retenue (38) ;
dans lequel l'élément interface (6) présente une première face (6A) orientée en direction du logement d'unité de commande (7) et une seconde face (6B) orientée en direction de l'ensemble brassard, dans lequel la première face est concave et est configurée pour suivre un corps cylindrique du logement d'unité de commande ; dans lequel est fournie une électrode d'ECG (32) disposée autour d'au moins une partie du logement d'unité de commande (7) et l'élément interface (6) est configuré pour fixer solidement l'électrode d'ECG sur le logement d'unité de commande ; et
dans lequel le raccord électrique comprend un câble de branchement raccordant l'électrode de contact au logement d'unité de commande.

2. Dispositif selon la revendication 1, dans lequel l'élément interface (6) est une pièce distincte interposée entre le logement d'unité de commande et l'ensemble brassard pour les fixer l'un à l'autre.

3. Dispositif selon la revendication 2, dans lequel l'élément interface est vissé sur le logement d'unité de commande.

4. Dispositif selon l'une quelconque des revendications 1 à 3, dans lequel les éléments de retenue sont formés comme des crochets (38).

5. Dispositif selon la revendication 4, dans lequel sont fournis deux crochets espacés l'un de l'autre d'une distance (E3) d'au moins 5 cm, ou d'au moins 50 % de la hauteur de l'ensemble brassard.

6. Dispositif selon l'une quelconque des revendications 1 à 5, dans lequel sont fournis des éléments/broches de verrouillage (138) dans des trous dans la direction transversale (T), et l'élément interface (6) comprend des trous transversaux (168) agencés dans la direction transversale (T) pour recevoir les broches de verrouillage (138).

7. Dispositif selon l'une quelconque des revendications 1 à 6, dans lequel l'armature de brassard (3) est une pièce en plastique élastique avec des éléments de retenue ou des crochets en formant partie intégrante.

8. Dispositif selon l'une quelconque des revendications 1 à 7, dans lequel l'élément interface est une pièce plate allongée, une pièce en plastique, ayant de préférence une épaisseur (6T) dans la direction d'assemblage (R) inférieure à 6 mm.

9. Dispositif selon l'une quelconque des revendications 1 à 8, dans lequel est fourni dans une partie intermédiaire de l'élément interface au moins un trou pour un branchement par câble électrique et au moins un trou débouchant (64a, 64b) pour une tuyauterie pour fluide pneumatique.

10. Procédé d'assemblage pour assembler un dispositif de surveillance de type à brassard selon la revendication 6, pour collecter des données cardiovasculaires concernant un utilisateur individuel, le procédé comprenant :
- la fourniture du logement d'unité de commande (7) incluant au moins une pompe,
- la fourniture de l'ensemble brassard (10) incluant l'armature de brassard élastique (3) et
- la fourniture de l'élément interface (6)
- la fixation de l'élément interface au logement d'unité de commande, par vissage
- l'assemblage de l'ensemble brassard à l'élément interface, par insertion des deux éléments de retenue dans les deux éléments de retenue complémentaires,
- la fixation de l'ensemble brassard à l'élément interface par insertion des éléments de verrouillage (138).

11. Procédé d'assemblage selon la revendication 10, comprenant en outre :
- le branchement électrique d'une fiche agencée avec l'ensemble brassard dans une prise agencée dans le logement d'unité de commande, avant l'assemblage de l'ensemble brassard à l'élément interface.

12. Procédé d'assemblage selon la revendication 10, dans lequel l'élément interface est fixé au logement d'unité de commande par vissage.
